# EUROPÄISCHE PATENTANMELDUNG

(11) **EP 0 873 728 A2**
(43) Veröffentlichungstag der Anmeldung: **28.10.1998**
(21) Anmeldenummer: 98106103.9
(22) Anmeldetag: 03.04.1998
(51) Int. Cl.: A61F 2/06

(54) **Radial aufweitbare Stützvorrichtung II**

(30) Priorität: 25.04.1997 DE 19717476
(71) Anmelder: W.C. Heraeus GmbH, D-63450 Hanau (DE)
(72) Erfinder: Gorywoda, Marek Dr., 63452 Hanau (DE); Herklotz, Günter Dr., 63486 Bruchköbel (US); Trötzschel, Jens, 63486 Bruchköbel (DE)
(74) Vertreter: Kühn, Hans-Christian

(57) **Zusammenfassung**

Die Erfindung betrifft eine radial aufweitbare Stützstruktur zur Offenhaltung von Lumina innerhalb eines Körpers, insbesondere eines Blutgefäßes, die einen rohrförmigen Körper umfaßt mit einer sich zwischen einem ersten und einem zweiten Ende erstreckenden Wandfläche, die aus langgestreckten, miteinander verbundenen Gliedern gebildet ist, mit einer ersten Gruppe von Gliedern, die sich im wesentlichen in Längsrichtung des rohrförmigen Körpers erstrecken, wobei jeweils benachbarte Glieder dieser ersten Gruppe paarweise unter Bildung eines einen Schlitz umfassenden Gliederpaares an ihren Enden miteinander verbunden sind, wobei diese Gliederpaare mit in Umfangsrichtung des rohrförmigen Körpers benachbart angeordneten Gliederpaaren etwa in der Mitte ihrer Längsausdehnung unter Bildung eines die Längsachse des rohrförmigen Körpers umlaufenden Ringes verbunden sind und wobei mehrere miteinander verbundene Ringe entlang der Längsachse des rohrförmigen Körpers angeordnet sind.

Um eine Stützstruktur bereitzustellen, die während der Aufweitung praktisch keine Längenänderung erfährt, sind die Ringe untereinander mit langgestreckten, im wesentlichen in Längsrichtung des rohrförmigen Körpers verlaufenden Gliedern einer zweiten Gruppe von Gliedern verbunden, wobei benachbarte Schlitze eines Ringes durch Verbindungsstellen miteinander verbunden sind und die Glieder der zweiten Gruppe von Gliedern an ihren Enden jeweils mit einer Verbindungsstelle des Ringes verbunden sind.

## Beschreibung

Die Erfindung betrifft eine radial aufweitbare Stützstruktur zur Offenhaltung von Lumina innerhalb eines Körpers, insbesondere eines Blutgefäßes, die einen rohrförmigen Körper umfaßt mit einer sich zwischen einem ersten und einem zweiten Ende erstreckenden Wandfläche, die aus langgestreckten, miteinander verbundenen Gliedern gebildet ist, mit einer ersten Gruppe von Gliedern, die sich im wesentlichen in Längsrichtung des rohrförmigen Körpers erstrecken, wobei jeweils benachbarte Glieder dieser ersten Gruppe paarweise unter Bildung eines einen Schlitz umfassenden Gliederpaares an ihren Enden miteinander verbunden sind, wobei diese Gliederpaare mit in Umfangsrichtung des rohrförmigen Körpers benachbart angeordneten Gliederpaaren etwa in der Mitte ihrer Längsausdehnung unter Bildung eines die Längsachse des rohrförmigen Körpers umlaufenden Ringes verbunden sind und wobei mehrere miteinander verbundene Ringe entlang der Längsachse des rohrförmigen Körpers angeordnet sind.

Derartige Stützstrukturen sind beispielsweise aus aus EP 335 341 B1 bekannt. Hier sind Stützstrukturen beschrieben, die aus langgestreckten Gliederpaaren gebildet sind. Diese Stützstrukturen werden in verengte Blutgefäße oder andere ein Lumen aufweisende Körperdurchgänge eingeschoben, um diese nach Erweiterung durch eine Ballondilatation offen zu halten. Dabei werden die Stützstrukturen in ihrem Durchmesser aufgeweitet und sie verkürzen sich während der Aufweitung. Derartige Verkürzungen sind allerdings in der Regel unerwünscht, da diese Verkürzung dazu führt, daß eine wesentlich längere Stützstruktur in die Körperöffnung eingeführt werden muß, als sie am Einsatzort unmittelbar erforderlich ist. Die bekannten Strukturen passen sich Bögen oder Kurven in den Körperöffnungen relativ schlecht oder gar nicht an, so daß zusätzliche Biegungselemente vorgesehen werden müssen. Die bekannten Stützstrukturen weisen starre, rohrförmige Abschnitte auf, die durch gelenkige Verbindungen biegbar miteinander verbunden sind. In der Praxis hat es sich gezeigt, daß in diesen gelenkigen Bereichen, bedingt durch die Dauerunruhe im Gewebelager, Gewebshypertrophien entstehen können.

Andere bekannte Strukturen weisen eine ausgeprägte Verkürzung bei der Dehnung auf. Auch spiralige Strukturen sind bekannt. Diese haben allerdings ein für den Einsatz ungünstiges Verhalten an ihren Enden.

Aufgabe der vorliegenden Erfindung ist es, eine radial aufweitbare Stützstruktur zu schaffen, die während ihrer Aufweitung praktisch keine Längenverkürzung erfährt.

Die Aufgabe wird für die eingangs genannte Stützstruktur dadurch gelöst, daß die Ringe untereinander mit langgestreckten, im wesentlichen in Längsrichtung des rohrförmigen Körpers verlaufenden Gliedern einer zweiten Gruppe von Gliedern verbunden sind, wobei benachbarte Schlitze eines Ringes durch Verbindungsstellen miteinander verbunden sind und die Glieder der zweiten Gruppe von Gliedern an ihren Enden jeweils mit einer Verbindungsstelle des benachbarten Ringes verbunden sind. Bei der Aufweitung der Stützstruktur werden die Schlitze verbreitert und in Umfangsrichtung gedehnt. Die außerhalb der Schlitze angeordneten und in Längsrichtung der Stützstruktur gerichteten Glieder der zweiten Gruppe von Gliedern sind von dieser Verformung vollkommen entkoppelt. Sie behalten dabei ihre Form. Dadurch wird die Form der Stützstruktur während der Aufweitung nicht verkürzt, da die Länge der Stützstruktur ausschließlich von der Länge der Glieder der zweiten Gruppe von Gliedern bestimmt ist. Die hiervon einzige Ausnahme besteht an den beiden Enden der Stützstruktur, da diese Enden durch die Gliederpaare der ersten Gruppe von Gliedern gebildet werden und diese Gliederpaare bei Aufweitung der Schlitze verkürzt werden. Diese Verkürzung ist allerdings in Bezug auf die Gesamtlänge der Stützstruktur praktisch ohne Bedeutung. Durch eine in Längsrichtung erfolgende Weiterführung der Glieder der zweiten Gruppe von Gliedern bis unmittelbar an die Enden der Stützvorrichtung heran läßt sich auch die geringste Längenänderung bei Ausdehnung vermeiden. Vorzugsweise sind die Enden der Gliederpaare abgerundet. Die Glieder der ersten Gruppe von Gliedern, die diese Gliederpaare bilden, können bogenförmig ausgebildet sein, so daß die Gliederpaare ovale bzw. elliptische Schlitze bilden. Die Gliederpaare können auch in anderer Form, beispielsweise als Rechtecke oder Rauten ausgebildet sein.

Zweckmäßig ist es, daß die Glieder der zweiten Gruppe von Gliedern mindestens teilweise nicht geradlinig ausgebildet sind. Insbesondere können diese Glieder mindestens teilweise spiralförmig, wellenförmig (sinusoidal) oder mäanderförmig ausgebildet sein. Durch eine derartige Ausbildung sind diese Glieder der zweiten Gruppe in Längsrichtung der Stützstruktur dehnbar, so daß durch eine längs des Umfanges der Stützstruktur unterschiedliche Dehnung eine Biegung der gesamten Struktur in sich selbst erfolgt. Dabei sind die Längsdehnung und die Aufweitung funktionell vollkommen voneinander entkoppelt, verlaufen also unabhängig voneinander. Die Glieder der zweiten Gruppe von Gliedern können derart ausgebildet sein, daß die Amplitude der Wellenform sich längs dieser Glieder ändert. Beispielsweise kann die Amplitude zu den Verbindungsstellen hin, die als Stege ausgebildet sein können, abnehmen, so daß sie in der Mitte der Glieder am größten ist. Die Stützstruktur kann eine biokompatible Beschichtung aufweisen, wie sie beispielsweise aus EP 335 341 B1 bekannt ist. Die rohrförmigen Körper sind aus nahtlosen Rohren gebildet, um Verspannung zu vermeiden. Die Strukturen (Anordnung der Glieder) sind durch Laserschneiden, Elektroerosion, Ätzen oder auch Spanabhebend hergestellt.

Weitere vorteilhafte Ausgestaltungen der Erfindung sind in den Unteransprüchen angegeben.

Nachfolgend werden Ausführungsbeispiele der Erfindung anhand einer Zeichnung erläutert. In der Zeichnung zeigt
- Figur 1: eine Stützstruktur mit geradlinig ausgebildeten Gliedern der zweiten Gruppe,
- Figur 2: eine Stützstruktur mit teilweise wellenförmig ausgebildeten Gliedern der zweiten Gruppe und
- Figur 3: eine Stützstruktur, bei der die Enden geöffnete Schlitze aufweisen.

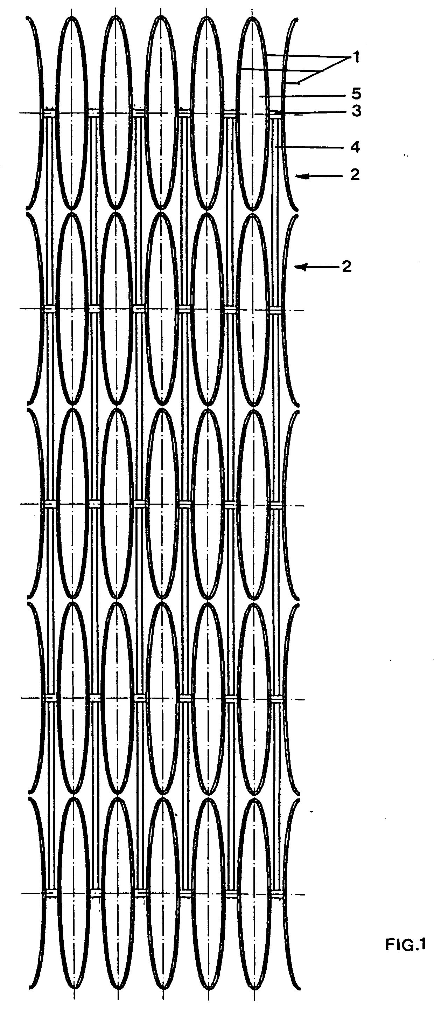

Figur 1 zeigt die abgerollte Wandfläche einer Stützvorrichtung. Bei dieser Stützvorrichtung sind als Aufweitungs- oder Dehnungselemente ovale Gliederpaare um den Umfang der Stützstruktur herum angeordnet. Dabei sind gekrümmte, sich in Längsrichtung erstreckende Glieder 1 paarweise zu solchen Gliederpaaren verbunden, wobei die Gliederpaare Schlitze 5 bilden, die die Form einer Ellipse aufweisen. Jeweils sechs dieser Gliederpaare sind um den Umfang der Stützstruktur herum angeordnet und bilden dabei jeweils diesen Umfang umlaufende Ringe 2. Dabei sind die einzelnen Gliederpaare durch als Stege ausgebildete Verbindungsstellen 3 miteinander verbunden. Diese Verbindungsstellen 3 sind in der Mitte der Längsausdehnung der Gliederpaare angeordnet, so daß diese sich gleichmäßig verformen können. In Längsrichtung übereinander bzw. hintereinander angeordnete Verbindungsstellen sind durch Glieder 4 einer zweiten Gruppe von Gliedern verbunden, wobei diese Glieder 4 an ihren Enden jeweils mit einer Verbindungsstelle 3 verbunden sind. Diese Glieder 4 sind als gerade Stäbe ausgebildet. Die Stützstruktur ist aus einem für medizinsiche Zwecke geeigneten Edelstahl gebildet.

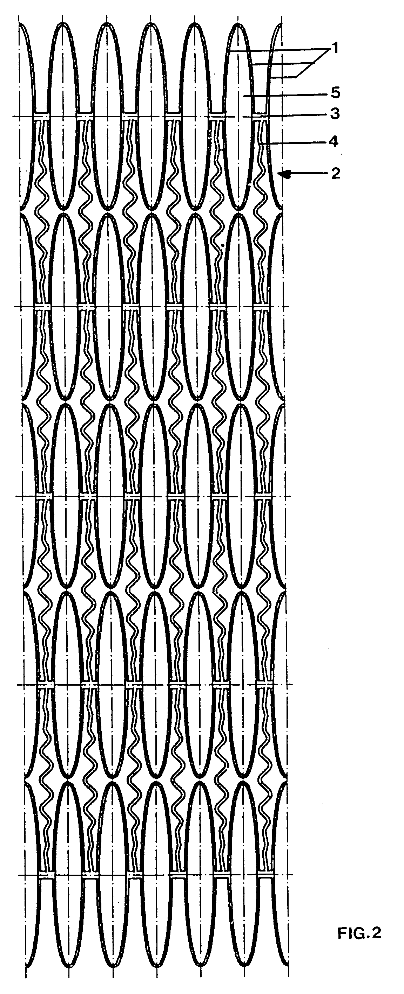

In Figur 2 ist eine ähnliche Stützstruktur dargestellt. Bei dieser Struktur sind die Glieder 4 der zweiten Gruppe allerdings teilweise wellenförmig ausgebildet, so daß sie als Dehnungsglieder innerhalb der Stützstruktur selbst wirken.

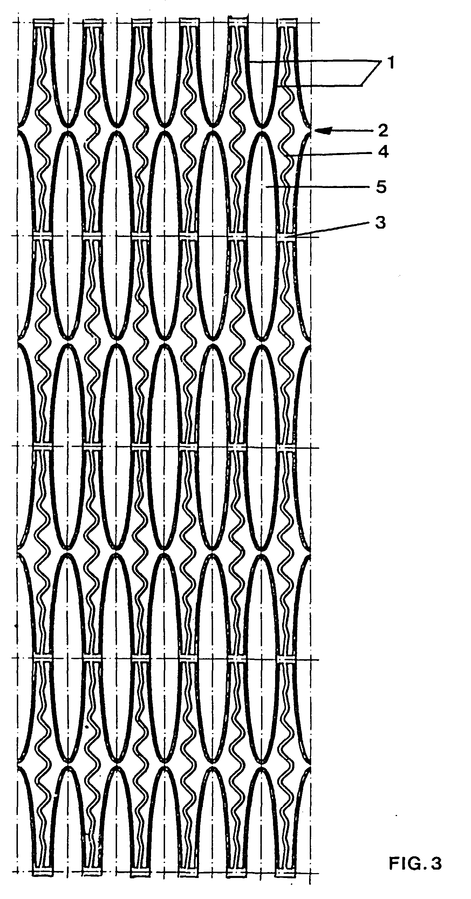

In Figur 3 ist eine der Stützstruktur in Figur 2 ähnliche Stützstruktur dargestellt. Allerdings sind die Enden der Stützstruktur als geöffnete Schlitze ausgebildet, was ein anderes Verhalten während des Aufweitens zur Folge hat, da die Ringe 2 hier als offene Strukturen ausgebildet sind. Diese Stützstruktur ist in sich sehr flexibel.

## Patentansprüche

1. Radial aufweitbare Stützstruktur zur Offenhaltung von Lumina innerhalb eines Körpers, insbesondere eines Blutgefäßes, die einen rohrförmigen Körper umfaßt mit einer sich zwischen einem ersten und einem zweiten Ende erstreckenden Wandfläche, die aus langgestreckten, miteinander verbundenen Gliedern gebildet ist, mit einer ersten Gruppe von Gliedern, die sich im wesentlichen in Längsrichtung des rohrförmigen Körpers erstrecken, wobei jeweils benachbarte Glieder dieser ersten Gruppe paarweise unter Bildung eines einen Schlitz umfassenden Gliederpaares an ihren Enden miteinander verbunden sind, wobei diese Gliederpaare mit in Umfangsrichtung des rohrförmigen Körpers benachbart angeordneten Gliederpaaren etwa in der Mitte ihrer Längsausdehnung unter Bildung eines die Längsachse des rohrförmigen Körpers umlaufenden Ringes verbunden sind und wobei mehrere miteinander verbundene Ringe entlang der Längsachse des rohrförmigen Körpers angeordnet sind, dadurch gekennzeichnet, daß die Ringe (2) untereinander mit langgestreckten, im wesentlichen in Längsrichtung des rohrförmigen Körpers verlaufenden Gliedern (4) einer zweiten Gruppe von Gliedern verbunden sind, wobei benachbarte Schlitze (5) eines Ringes (2) durch Verbindungsstellen (3) miteinander verbunden sind und die Glieder (4) der zweiten Gruppe von Gliedern durch Verbindungsstellen (3) an ihren Enden jeweils mit einer Verbindungsstelle (3) des benachbarten Ringes (2) verbunden sind.

2. Radial aufweitbare Stützstruktur nach Anspruch 1, dadurch gekennzeichnet, daß die Enden der Gliederpaare abgerundet sind.

3. Radial aufweitbare Stützstruktur nach Anspruch 1 oder 2, dadurch gekennzeichnet, daß die Verbindungsstellen (3) als Stege ausgebildet sind.

4. Radial aufweitbare Stützstruktur nach einem der Ansprüche 1 bis 3, dadurch gekennzeichnet, daß die Gliederpaare als Ellipsen ausgebildet sind.

5. Radial aufweitbare Stützstruktur nach einem der Ansprüche 1 bis 4, dadurch gekennzeichnet, daß die Glieder (4) der zweiten Gruppe mindestens teilweise nichtgeradlinig ausgebildet sind.

6. Radial aufweitbare Stützstruktur nach Anspruch 5, dadurch gekennzeichnet, daß die Glieder (4) der zweiten Gruppe mindestens teilweise spiralförmig, wellenförmig oder mäanderförmig ausgebildet sind.

7. Radial aufweitbare Stützstruktur nach einem der Ansprüche 1 bis 6, dadurch gekennzeichnet, daß um den Umfang des rohrförmigen Körpers herum mindestens vier einen Schlitz bildende Gliederpaare benachbart zueinander angeordnet sind.

8. Radial aufweitbare Stützstruktur nach einem der Ansprüche 1 bis 7, dadurch gekennzeichnet, daß sie im wesentlichen aus einem oder mehreren Metalle der Gruppe Tantal, Titan, Niob, Stahl, Platin oder einer Legierung mindestens eines dieser Metalle mit mindestens einem weiteren Metall gebildet ist.

9. Radial aufweitbare Stützstruktur nach einem der Ansprüche 1 bis 8, dadurch gekennzeichnet, daß sie mit einem biokompatiblen Material beschichtet ist.
